**Europäisches Patentamt**

**European Patent Office**

**Office européen des brevets**

(19)

(11) Veröffentlichungsnummer : **0 358 600 B1**

(12) **EUROPÄISCHE PATENTSCHRIFT**

(45) Veröffentlichungstag der Patentschrift :
**15.04.92 Patentblatt 92/16**

(51) Int. Cl.⁵ : **A61F 2/34**

(21) Anmeldenummer : **89810532.5**

(22) Anmeldetag : **13.07.89**

(54) **Künstliche Hüftgelenkspfanne.**

(30) Priorität : **25.08.88 CH 3168/88**

(43) Veröffentlichungstag der Anmeldung :
**14.03.90 Patentblatt 90/11**

(45) Bekanntmachung des Hinweises auf die
Patenterteilung :
**15.04.92 Patentblatt 92/16**

(84) Benannte Vertragsstaaten :
**AT DE FR GB IT**

(56) Entgegenhaltungen :
**EP-A- 0 058 753**
**EP-A- 0 237 751**
**FR-A- 2 184 159**
**FR-A- 2 272 637**

(56) Entgegenhaltungen :
**MED.-ORTHOP.-TECHN., Band 106, Nr. 6, No-**
**vember/Dezember 1986, Seiten 194-197; M.**
**UNGETHÜM et al.: "Biomechanische Aspekte**
**zementfreier Hüftpfannen-Implantate mit**
**Schraubverankerung"**

(73) Patentinhaber : **GEBRÜDER SULZER**
**AKTIENGESELLSCHAFT**
**Zürcherstrasse 9**
**CH-8401 Winterthur (CH)**

(72) Erfinder : **Stühmer, Karl-Gerhart, Dr.-med.**
**St. Elisabethen-Krankenhaus**
**W-7980 Ravensburg (DE)**
Erfinder : **Koch, Rudolf**
**Oberdorfstrasse 229**
**CH-8267 Berlingen (CH)**
Erfinder : **Frey, Otto, Dr.**
**Wallrütistrasse 56**
**CH-8400 Winterthur (CH)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine künstliche Hüftgelenkspfanne zur zementfreien Verankerung im menschlichen Körper, bestehend aus aus einem Pfannenkörper und einer den Pfannenkörper aufnehmenden halbkugelförmigen Aussenschale aus Reintitan oder einer Titanlegierung, deren äussere Oberfläche mit einem selbstschneidenden Schraubengewinde konstanter Steigung mit durch Spannuten unterbrochenen Zähnen versehen ist, für dessen Gewindegänge gilt, daß die Spitzenbreiten (a) kleiner als die Fußbreiten (A) sind.

Ein Hüftgelenkspfanne der vorstehend genannten Art ist bekannt aus der EP-A-237 751. Durch die Einhaltung der vorstehend genannten Bemessungsvorschriften ergibt sich mit dieser selbstschneidenden Hüftgelenkspfanne als Folge der relativ umfangreichen Knochenverdichtung durch das keilförmige Gewinde ein besonders stabiler und vor allem auch gegen unbeabsichtigte Rotation widerstandfähiger Sitz bei zementfreier Verankerung der Pfanne im Knochen. In der Praxis hat sich jedoch herausgestellt, dass das mit dem Schneiden des Gewindes verbundene Implantieren der Pfanne dem Operateur erhebliche Schwierigkeiten bereitet, da das keilförmige Gewinde beim Schneiden sehr rasch zu klemmen beginnt.

Aufgabe der vorliegenden Erfindung ist es, die bekannte Konstruktion so zu verbessern, dass die Arbeit des Operateurs erleichtert wird.

Diese Aufgabe wird mit der Erfindung dadurch gelöst, dass an der Spitze die Gewindegänge auf 1/3 ihrer Länge mit der konstanten Dicke der Spitzenbreite versehen sind.

Das erste Drittel eines Gewindeganges bewirkt vor allem ein erleichtertes Gewindeschneiden, während der feste Sitz durch die letzten zwei Drittel gewährleistet wird. Wie bei der bekannten Konstruktion hat es sich als vorteilhaft erwiesen, wenn die Steigung des Gewindes z.B. 6,5 mm beträgt.

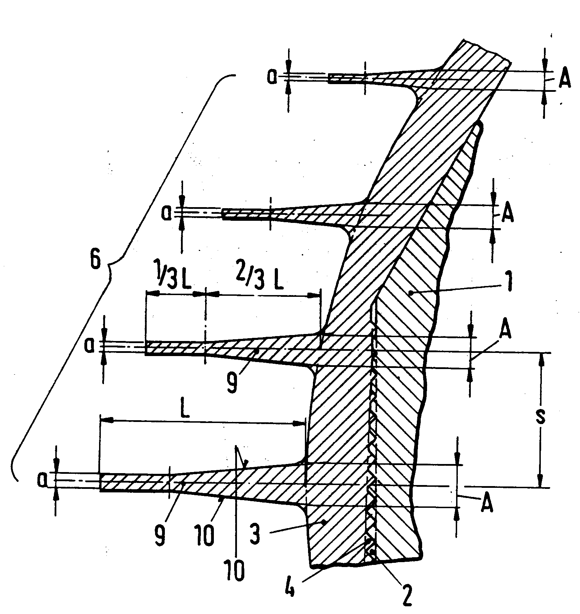

Im folgenden wird die Erfindung anhand eines Ausführungsbeispiels im Zusammenhang mit der Zeichnung näher erläutert.

Die einzige Figur zeigt einen Meridianschnitt durch den äquatorialen Bereich der neuen Pfanne in gegenüber natürlicher Grösse stark vergrösserter Darstellung.

Die Hüftgelenkspfanne nach dem Ausführungsbeispiel ist zweiteilig und weist einen Pfannenkörper 1 auf, dessen äussere Begrenzung im Querschnitt ein Vieleck bildet; die äquatorialen Seiten dieses Vielecks sind mit einem Gewinde 2 versehen. Ueber das Gewinde 2 und ein entsprechendes Gegengewinde 4 wird der Pfannenkörper 1 im an seine Aussenbegrenzung angepassten Hohlraum einer metallischen Aussenschale 3 fixiert.

Von der Seite des Aequators her ist in den Pfannenkörper 1 eine nicht gezeigte eigentliche Pfannenschale in Form einer hohlen Halbkugel eingearbeitet, die den Gelenkkopf einer ebenfalls nicht dargestellten Femurkopfprothese aufnimmt.

Die äussere halbkugelförmige Oberfläche, mit der die Aussenschale 3 im Beckenknochen verankert wird, trägt in ihren Bereichen äquatornaher und mittlerer Breiten in bekannter Weise ein mehrgängiges Schraubengewinde 6 mit konstanter Steigung s.

Die Länge L der Gewindegänge 9 des Schraubengewindes 6, die in Umfangsrichtung durch jeweils in Meridianrichtung der Halbkugel verlaufenden, nicht gezeigte Spannuten unterteilt sind, nimmt, im gezeigten Ausführungsbeispiel, beim Fortschreiten vom Aequator zum Pol kontinuierlich ab. Die Länge L ist dabei, in der Mittelebene jedes Gewindeganges gemessen, der Abstand zwischen der Halbkugeloberfläche und der Spitze des Gewindeganges 9. Sie beträgt in Absolutwerten bei Aussenschalen aus Titan oder Titanlegierungen 3 bis 8 mm.

Die Grösse A, d.h. die Dicke eines Gewindeganges an seinem Fusspunkt, ergibt sich aus den - an sich durch einen hohlkehlenartigen Radius abgerundeten - Ecken, die die Schnittpunkte der verlängerten Flanken 10 eines Gewindeganges 9 mit der Halbkugeloberfläche der Aussenschale 3 bilden, während die Dicke der einzelnen Gewindegänge 9 an ihrer Spitze mit a bezeichnet ist.

Erfindungsgemäss ist das vordere Drittel der Länge L der Gewindegänge 9 mit parallel verlaufenden Flanken versehen, die die Dicke der Spitzenbreite a haben. Dieses vordere Drittel bewirkt ein gegenüber der bekannten Konstruktion erheblich erleichtertes Schneiden des Gewindes, da in seinem Bereich ein Klemmen der Gewindegänge 9 im Knochen nicht auftritt.

Die Gewindegänge 9 werden bei der Fabrikation der Aussenschale 3 beispielsweise durch numerisch gesteuertes Gewindeschneiden oder durch Fräsen aus dem Vollen eines Rohlings herausgearbeitet.

## Patentansprüche

1. Künstliche Hüftgelenkspfanne zur zementfreien Verankerung im menschlichen Körper, bestehend aus einem Pfannenkörper (1) einer den Pfannenkörper (1) aufnehmenden, halbkugelförmigen Aussenschale (3) aus Reintitan oder einer Titanlegierung, deren äussere Oberfläche mit einem selbstschneidenden Schraubengewinde (6) konstanter Steigung mit durch Spannuten unterbrochenen Zähnen versehen ist, für dessen Gewindegänge (9) gilt, daß die Spitzenbreiken (a) Kleiner als die Fußbreiten (A) sind, dadurch gekennzeichnet, daß für die Gewindegänge (9), bei Absolutwerten für ihre Länge (L) von 3 mm $\leq$ L $\leq$ 8 mm, gilt: 0,25 L $\leq$ Fussbreite (A) $\leq$ 0,7 L, sowie 0,1 L $\leq$ Spitzenbreite (a) $\leq$ 0,4 L, und dass an der Spitze die Gewindegänge (9) auf 1/3 ihrer Länge (L) mit der konstanten Dicke der Spitzenbreite (a) versehen sind.

2. Hüftgelenkspfanne nach Anspruch 1, dadurch gekennzeichnet, dass die Steigung (s) des Gewindes (6) 6,5 mm beträgt.

**Claims**

1. A prosthetic acetabulum for uncemented fixing in the human body and comprising a socket (1) and, receiving the same, a hemispherical outer shell (3) which is made of pure titanium or a titanium alloy and whose outside surface is formed with a self-tapping constant-pitch screwthread (6) having teeth interrupted by gashings, the turns (9) of the screwthread (6) being subject to the condition that the crest widths (a) are smaller than the root widths (A), characterised in that the screwthread turns (9) are subJect, in absolute values for their length (L) of 3 mm $\leq$ L $\leq$ 8 mm, the condition that 0.25 L $\leq$ root width (A) $\leq$ 0.7 L and 0.1 L $\leq$ crest width (a) $\leq$ 0.4 L, and at their apexes the screwthread turns (9) have over one-third of their length (L) the constant thickness of the crest width (a).

2. An acetabulum according to claim 1, characterised in that the pitch (s) of the screwthread (6) is 6.5 mm.

**Revendications**

1. Cavité cotyloïde artificielle destinée à être fixée sans ciment dans le corps humain, se composant d'un corps de cavité (1) et d'une coquille extérieure hémisphérique (3) logeant le corps (1) de la cavité, réalisée en titane pur ou en alliage de titane et dont la surface extérieure comporte un filetage autotaraudeur (6) ayant un pas constant et dont les dents sont interrompues par des rainures à copeaux, et de plus dont les filets (9) ont au sommet des largeurs (a) inférieures aux largeurs (A) à la base, caractérisée en ce que les filets (9), dont les longueurs (L) ont des valeurs absolues de 3 mm $\leq$ L $\leq$ 8 mm, satisfont aux relations : 0,25 L $\leq$ largeur à la base (A) $\leq$ 0,7 L, et 0,1 L $\leq$ largeur (a) au sommet $\leq$ 0,4 L et en ce que les filets (9) ont au sommet une épaisseur constante correspondant à la largeur au sommet (a) sur 1/3 de leur longueur (L).

2. Cavité cotyloïde selon la revendication 1, caractérisée en ce que le pas (s) du filetage (6) est de 6,5 mm.